# EUROPEAN PATENT SPECIFICATION

(11) **EP 2 616 876 B1**
(45) Date of publication and mention of the grant of the patent: **23.06.2021**
(21) Application number: 10857121.7
(22) Date of filing: 13.09.2010
(51) Int. Cl.: G02C 7/04, A61F 9/00, G02C 7/06, A61F 2/14

(54) **SYSTEM FOR RETARDING PROGRESSION OF MYOPIA**
SYSTEM ZUR VERZÖGERUNG DER PROGRESSION VON KURZSICHTIGKEIT
SYSTÈME DE RETARDEMENT DE LA PROGRESSION DE LA MYOPIE

(43) Date of publication of application: 24.07.2013
(73) Proprietor: The Hong Kong Polytechnic University, Hong Kong (CN)
(72) Inventor: TSE, Yan Yin, Hong Kong (CN); TO, Chi Ho, Hong Kong (CN); LAM, Siu Yin, Hong Kong (CN)
(74) Representative: Vogel, Andreas
(86) International application number: PCT/CN2010/076839
(87) International publication number: WO 2012/034265

(56) References cited:
- EP-A1- 1 967 892
- WO-A1-2007/041796
- CA-C- 2 263 635
- CN-A- 1 909 860
- CN-A- 101 467 092
- CN-A- 101 688 983
- US-A1- 2003 058 407
- US-A1- 2005 068 494
- US-A1- 2006 082 729
- US-A1- 2010 036 489
- None

## Description

### Technical Field

The present invention relates to a refractive lens for retarding the progression of myopia.

### Background of the Invention

Shortsightedness or myopia is a common refractive disorder of human eyes.
Objects beyond a distance from a myopic person are focused in front of the retina and is perceived as blurry images. Common myopia develops when the eye grows excessively longer than the combined focal length of the optical elements of the eye. Myopia usually progresses in human eyes over time and is typically managed by regularly renewed prescriptions of corrective spectacles and contact lens. Those
lenses provide clear vision but do not retard progression of myopia. Undesirable sight-threatening eye diseases are also associated with high myopia. Therefore, there is a need for new technology to reduce the economical and social burden produced by common myopia by providing clear vision and a retardation function at the same time. Recent scientific publications have stated that the dimensional
growth of developing eyes is modulated by optical defocus, which is resulted when images are projected away from the retina. Refractive development of the eye is influenced by the equilibrium between defocus of opposite directions. In particular, it has been documented that artificially induced "myopic defocus" (image projected in front of the retina) may retard myopia from progressing further.

US patent 7,025,460 [Smith] teaches a method to introduce peripheral (off-axis) myopic defocus relative to central (on-axis) focused image to control the progression of myopia. It teaches against projections of defocus on central retina to provide clear vision correction. The method may not be able to achieve optimal effectiveness for retarding myopia progression. A similar problem is evident in the disclosure of PCT application PCT/US2007/070419 [Holden]. Holden teaches that the peripheral optical zone producing defocus be substantially outside the normal pupil diameter of the patient. A similar problem is also evident in the disclosure of US patent application 60/584894 [Phillips]. Phillips suggests a non-concentric design to degrade optical quality.

WO 2006/034652 [To] teaches the use of concentric multi-zone bifocal lenses in which myopic defocus is induced both axially and peripherally for visual objects of all viewing distances. Those teachings have been shown to be effective in both animal study and human clinical trial for retarding myopia progression. However,
minor problems have been identified in the clinical trial and is waiting to be improved. The use of bifocal lens taught projects a secondary single homogeneous defocused image, which is sometimes perceived as a bright "ghost" image causing discomfort to the patient. In addition, the homogeneous secondary defocused image might mislead a minor portion of the patients to adjust their accommodation
habit and choose to focus with the secondary defocused image instead of the designated primary image and thus jeopardizing the retarding function. Similar problems are evident in US patent application 60/905821 [Phillips], in which a concentric multi-zone bifocal lens using the same principle was described as a form of contact lens.

US 6,045,578 [Collins] discloses a method manipulating spherical aberration for myopia control. In optical and mathematical terms, longitudinal aberration describes the extent where marginal rays are bent more than or less than the paraxial rays. It is different from optical defocus which describes the distance an image is away from the reference image plane (retina in eye). Unlike the case of defocus, it has not gained support from scientific literature and there is no identified report that manipulating spherical aberration has any effect on retarding myopia progression.

US 6,752,499 [Aller] teaches prescribing commercially available bifocal contact lenses to young myopic patients who also exhibit near point esophoria and accommodative lag to control myopia. Its effectiveness is questionable because a higher dose of undesirable hyperopic defocus is induced by the primary refractive power (distant zone) when it teaches to reduce the esophoria and accommodative
lag during near vision as it requires the wearer to switch their focus to use the second refractive power (near zone). In addition, it is not supported from more recent literature that esophoria is related to myopia progression.

EP 1 967 892 A1 discloses contact lenses and methods for preventing or slowing progression of myopia are described. The contact lenses include a vision correction area and a myopic defocus area. Various examples of the contact lenses are described. Methods include providing the present contact lenses, or applying one or more of the contact lenses to an eye or eyes of a person. The contact lenses and methods are particularly useful in children.

US 2005/0068494 A1 [Griffin] teaches multifocal and single focus lenses, which are defined by nonconical aspheric optical surfaces. Various alternative surface shapes provide one or more vision regions bounded by optical steps. Each optical step has rapidly and smoothly changing power in the radial direction which creates an induced aperture through which the cortical elements of the human vision system are induced to concentrate. The induced aperture results in increased clarity and enhanced vision.

US 2010/0036489 A1 [Lindacher] teaches a lens that is capable of preventing or slowing the progression of myopia when worn by a person. The lens has a power profile that reduces on-axis and off-axis hyperopic defocus created by the optics of the eye by creating on-axis and off-axis myopic defocus. The on-axis and off-axis myopic defocus is created by providing light rays that pass through a 20 central vision region of the optical portion and light rays that pass through a peripheral region of the optical portion an increase in positive (plus) power. The overall effect is to prevent or slow the progression of myopia without any perceptible degradation in the person's central vision.

WO 2007/041796 A1 [Varnas] discloses an ophthalmic lens element for correcting myopia in a wearer's eye is disclosed. The lens element includes a central zone and a peripheral zone. The central zone provides a first optical correction for substantially correcting myopia associated with the foveal region of the wearer's eye. The peripheral zone surrounds the central zone and provides a second optical correction for substantially correcting myopia or hyperopia associated with a peripheral region of the retina of the wearer's eye. A system and method for dispensing or designing an ophthalmic lens element for correcting myopia in a wearer's eye is also disclosed.

### Summary of the Invention

There is provided a refractive lens for retarding the progression of myopia in a human eye, the refractive lens comprising:
a concentric annular multi-zone refractive lens including:
   correcting zones of optical power for correcting refractive error, and
   defocusing zones having a progressive power profile for projecting multiple defocus images or non-homogenous defocused images in front of at least a part of retina to inhibit myopic eye growth, the defocusing zones having a range of less negative powers than the optical power for correcting refractive power of the correcting zones;
wherein the correcting zones and defocusing zones are alternated in the lens, wherein the lens has at least a total number of more than three correcting zones and defocusing zones alternating in a concentric manner,
wherein the central first correcting/defocusing zone is smaller than the pupil is measured under photopic lighting conditions,
wherein the same single homogeneous power is maintained over the correcting zones,
wherein the defocusing zones have a sinusoidal power profile.

The lens may be in the form of a contact lens and its optical surface includes 2 to 40 alternating correcting and defocusing zones.

The lens may have a central zone that is a circular first correcting zone having a diameter smaller than the pupil of the eye under photopic lighting.

The first correcting zone may be immediately surrounded by a first defocusing zone having an annular shape.

The first defocusing zone may be immediately surrounded by a second correcting zone having an annular shape.

The second correcting zone may be surrounded by additional defocusing zones and correcting zones in an alternating manner.

The lens may have a central zone that is a circular first defocusing zone having a diameter smaller than the pupil of the eye under photopic lighting.

The first defocusing zone may be immediately surrounded by a first correcting zone having an annular shape.

The first correcting zone may be immediately surrounded by a second defocusing zone having an annular shape.

The second defocusing zone may be surrounded by additional defocusing zones and correcting zones in an alternating manner.

The maximal difference between the power of the at least one correcting zone and
the peak power of the at least one defocusing zone may be between about 0.5 to 10.0 diopters.

The refractive lens may further comprise:
the at least one correcting zone focussing on visual objects at all distances for viewing purpose, and
an ocular accommodation for near viewing tasks.

The diameter of the central zone may be about 2.0mm to 4.5mm.

At least part of the at least one defocusing zone and at least a part of the correcting zone may overly the pupil at the same time.

The refractive lens may further comprise a range of powers for the at least one defocusing zone.

The power profile of the at least one defocusing zone may be progressive causing transitions between adjacent curves to become progressive.

The progressive power profile may be generated by adjusting the radius of curvature of the anterior refractive surface of the lens or by adjusting the posterior curvature or the refractive index of the lens.

The refractive lens may further comprise an integrated progressive transition curve between adjacent zones of the lens.

The refractive lens may further comprise a maintained single homogeneous power over the at least one correcting zone.

The refractive lens may have seven correcting zones and six defocusing zones to maintain a relatively stable area ratio between the correcting zones and defocusing zones for a range of pupil sizes and lighting conditions.

The refractive lens may have two correcting zones and one defocusing zone.

In a second aspect, not covered by the invention, there is provided a system for retarding the progression of myopia in a human eye, the system comprising:
a concentric annular multi-zone refractive lens including:
   at least one correcting zone of optical power for correcting refractive error, and
   at least one defocusing zone for projecting at least one non-homogenous defocused image in front of at least a part of retina to inhibit myopic eye growth, the at least one defocusing zone having at least one less negative power;
wherein the correcting and defocusing zones are alternated in the lens and the zones are connected to each other through integrated progressive transition
   curves.

In a third aspect, not covered by the invention, there is provided a method for retarding the progression of myopia in a human eye, the method comprising:
generating a focused image on a retina of the human eye to correct
   a
   refractive error of the eye and provide clear vision; and
at the same time, generating at least one non-homogenous defocused image in front of the central retina to create myopic defocus to retard the progression of myopia.

In a fourth aspect, not covered by the invention, there is provided a concentric multi-zone multifocal lens having a characteristic partial sinusoidal power profile, the lens comprising:
at least one correcting zone of optical power for correcting refractive error and
at least one defocusing zone for projecting at least one non-homogenous defocused image in front of at least a part of retina to inhibit myopic eye growth, the at least one defocusing zone having at least one less negative power;
wherein the correcting and defocusing zones are alternated in the lens and the zones are connected to each other through integrated progressive transition curves.

The method for treating progression of myopia in a human eye includes producing at least one focused image on a retina of the human eye and at least one non- homogenous defocused images (or referred to as heterogeneous defocused image) in front of the retina to generate myopic defocus.

The method for treating progression of myopia in a human eye includes providing a concentric annular multi-zone lens having correcting zones and defocusing zones.

The correcting zones provide a refractive power to correct the refractive error of the eye. The defocusing zones include a range of less negative refractive powers for generating defocus.

Preferably, the concentric annular multi-zone lens are provided in the form of contact lenses. In particular, contact lenses are highly suitable for myopic children and young adults whose pupils are relatively large.

The method for treating progression of myopia in a human eye includes generating defocus on the entire retina including both axial retina and peripheral retina in order to achieve maximal effectiveness.

The multi-zone lens of the present invention has both correcting zones and defocusing zones which overlie the pupil so that axial rays originated from objects in the central vision field are intercepted by both zones to project a sharp image on the retina and a defocused image or defocused images in front of the retina at the same time.

Experiments have shown that myopic defocus induced by using a concentric annular multi-zone lens effectively retards myopia progression in chickens, guinea pigs and human children. It is also desirable to maintain providing clear vision while eliminating the undesired visual disturbance caused by the secondary defocused image, which is made to be a single homogeneous image of high optical quality and brightness. In the present invention, to eliminate the undesired visual disturbance, the following techniques are possible:
(i) using a range of powers for the defocusing zones instead of a single power,
(ii) using a progressive (specifically sinusoidal) power profile for the defocusing zones,
(iii) using an integrated progressive transition curve between adjacent zones,
(iv) maintaining the same single homogeneous power over the correcting zones, and
(v) using a larger difference between the single power of the correcting zones and the peak power of the defocusing zones.

The progressive transition curve is integrated in the progressive power profile of the defocusing zones.

The progressive (specifically sinusoidal) power profile of the defocusing zones is preferably achieved by manipulating the radius of curvature of the anterior refractive surface of the lens. It is also possible to achieve a progressive power profile for each defocusing zone by manipulating the posterior curvature or the refractive index of the lens.

To ensure that the defocused images are formed on the central retina, at least part of the defocusing zone and at least a part of the correcting zone overlies the pupil at the same time. To provide this effect, the central first correcting/defocusing zone is smaller than the pupil as measured under photopic lighting condition. It has been found that the pupil sizes of the target population range between 4.0mm to 5.0mm under photopic conditions, and between 6.0mm to 7.8mm under dim lighting conditions. Although it is desirable for lenses to be custom-made for individuals using measured parameters, it is possible to estimate appropriate zone widths using population averages based on age and ethnicity to facilitate mass production of lenses.

### Brief Description of the Drawings

An example of the invention will now be described with reference to the accompanying drawings, in which:
Figure 1A is a frontal view of a 7-zone concentric multi-zone multi-defocusing contact lens in accordance with an embodiment of the present invention;
Figure 1B shows a desirable power profile across the lens in accordance with an embodiment of the present invention;
Figure 1C shows a less desirable power profile across a concentric multi-zone lens of the prior art;
Figure 2A is a frontal view of a 3-zone concentric multi-zone contact lens in accordance with an embodiment of the present invention;
Figure 2B shows a desirable power profile across the lens in accordance with an embodiment of the present invention;
Figure 2C shows a less desirable power profile across a concentric multi-zone lens of the prior art;
Figure 3A is a diagram of a myopic eye fitted with a concentric multi-zone multi- defocusing lens in accordance with an embodiment of the present invention; and
Figure 3B is a diagram of a myopic eye fitted with a concentric multi-zone bifocal lens of the prior art;
Figure 4 is a process flow diagram of a method for retarding the progression of myopia in a human eye in accordance with a preferred embodiment of the present invention.

### Detailed Description of the Drawings

Two examples for retarding progression of myopia in human eyes are described. The apparatuses used to practice this method alter the defocus equilibrium of the eye to influence dimensional eye growth in a direction towards emmetropia. In particular, myopic defocus is induced in the eye to retard the progression of myopia.

The myopic defocus can be introduced by various ways and apparatuses, for example, by spectacle lens, spectacle lens add-on, contact lens, corneal or intraocular implant. It is important that myopic defocus is introduced when normal vision is maintained throughout the treatment. That means that a focused homogenous image must be maintained at the central retina during the treatment.

A concentric bifocal lens splits incoming light rays and focuses them into two images. Therefore, it is an effective means for simultaneously providing clear vision and myopic defocus. An important next step is to reduce undesirable visual disturbance caused by the homogenous defocused image overlapping on the focused retina image while achieving optimal retarding effect.

A treatment method is provided to introduce multiple defocused images in front of the retina and a single homogeneous focused image on the retina. This is achieved using a concentric multi-zone multifocal lens having a characteristic partial sinusoidal power profile.

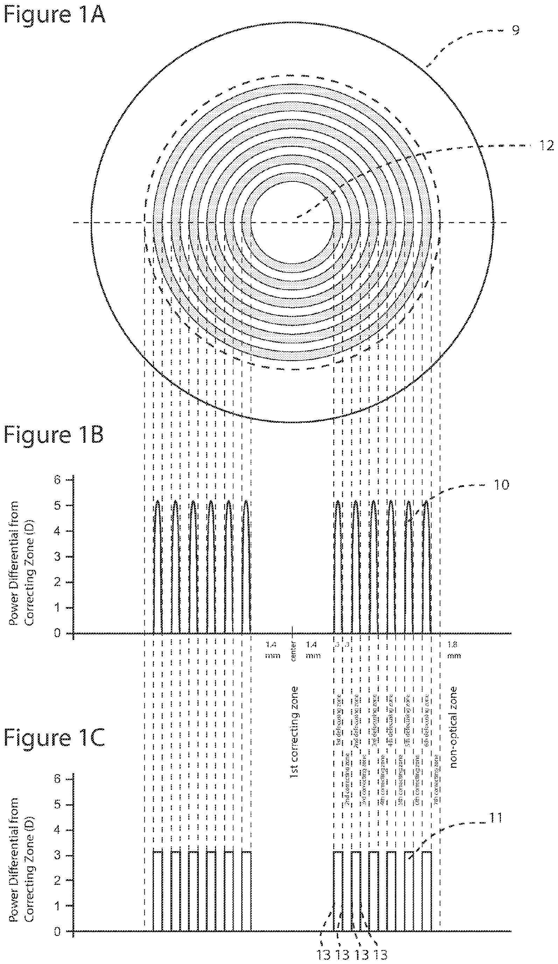

Referring to Figure 1A, a 13-zone contact lens 9 is provided as a concentric multi- zone multifocal lens having a characteristic partial sinusoidal power profile. There are seven correcting zones and six defocusing zones alternating in a concentric manner. This number of alternating zones allows a reasonably stable area ratio between the two zones to be maintained under a range of pupil sizes and lighting conditions. Therefore stable clear vision and retarding effectiveness can be maintained in different lighting conditions. A central correcting zone 12 has a diameter of 2.8mm, which is smaller than the typical pupil size of children and young adults (4.0-5.0mm). Therefore, a few consecutive annular defocusing and correcting zones always overlies on the pupil and introduce a defocused image on the retina including the central on-axis region. The width of each annular zone of alternating functions is 0.3mm. All seven correcting zones share the same power to neutralize the pre-existing myopia (for example, distance prescription) of a wearer. For example, if the wearer has 3D of myopia, the power of the correcting zone may be -3D. The homogenous power profile in the correcting zones ensure that the image formed is homogenous and of high optical quality for good vision. On the other hand, each of the six defocusing zones comprises a range of less negative powers. The progressive (specifically sinusoidal) power profile 10 of the defocusing zones does not introduce a homogenous secondary defocused image but non- homogenous multiple defocused images that are slightly separated from each others and of lower intensity. This is in contrast to a square power profile 11 of the lens of the prior art,

The 5D power differences between correcting zones and defocusing zones ensure that the defocus generated is potent enough to achieve optimal retarding effect. For example the power of each of the defocusing zone ranges between -3 to and 2D to introduce myopic defocus, if the wearer has 3D of myopia. An extra benefit from the sinusoidal power profile of the defocusing zones is that the transitions between adjacent curves become progressive and do not create undesirable diffraction as created by the sudden power change 13 in the square power profile of prior art designs. A progressive transition curve occurs where the power change across adjacent zone is continuous. There are many possible shapes not covered by the invention which can be regarded as progressive transition including but not limited to: sigmoidal, polynomial, conical, parabolic. In the described embodiment, the method is described using a partial sinusoidal shape for the overall power profile of the lens.

The edge of the lens 9 is made of a non-optical zone for lens stabilization purpose. The total diameter of the lens 9 in the example is 13.6mm. It is preferred that the alternating sinusoidal power profile be achieved by manipulating the radius of curvature of the anterior surface of the lens 9, and leaving the posterior surface for toric manipulations to correct astigmatism.

In Figure 1B, the power profile of each defocusing zone is progressive and is like a sinusoidal pattern. In contrast, in Figure 1C, the prior art shows that the power profile of each defocusing zone is homogenous, and that this square power profile
of the lens causes a sudden power change 13.

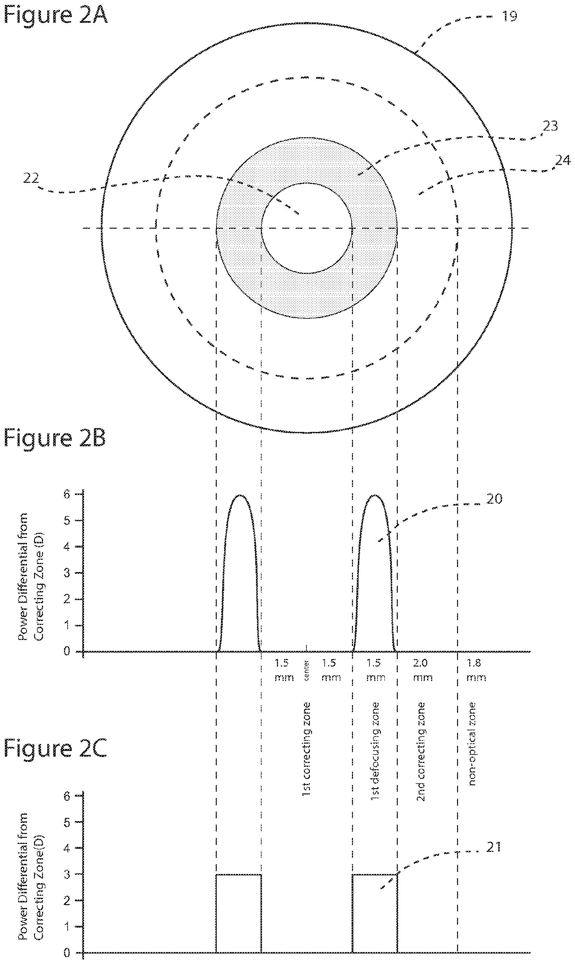

Referring to Figure 2A, a 3-zone contact lens 19 is provided as a concentric multi- zone multifocal lens having a characteristic partial sinusoidal power profile. There are two correcting zones and one defocusing zone alternating in a concentric manner. This number of alternating zones reduces manufacturing complexity yet still provides capability to introduce non-homogenous multiple defocused images covering the entire retina.

The central correcting zone 22 has a diameter of 3mm, which is smaller than the typical pupil size of children and young adults (4.0mm to 5.0mm). Therefore, the immediate surrounding annular first defocusing zone 23 always overlies with the pupil, introducing a defocused image on the retina (including the central on-axis region of the retina). The defocusing zone 23 has an annular width of 1.5mm and is surrounding by a second correcting zone 24 having an annular width of 2.0mm. Again, both of the two correcting zones 22, 24 share the same homogeneous power to neutralize the pre-existing myopia (for example, distance prescription) of the wearer. For example if the wearer has 4D of myopia, the power of the correcting zones 22, 24 may be -4D. The homogenous power profile in the correcting zones 22, 24 ensure that the image formed is homogenous and of high optical quality for good vision. On the other hand, the defocusing zone 23 comprises a range of less negative powers.

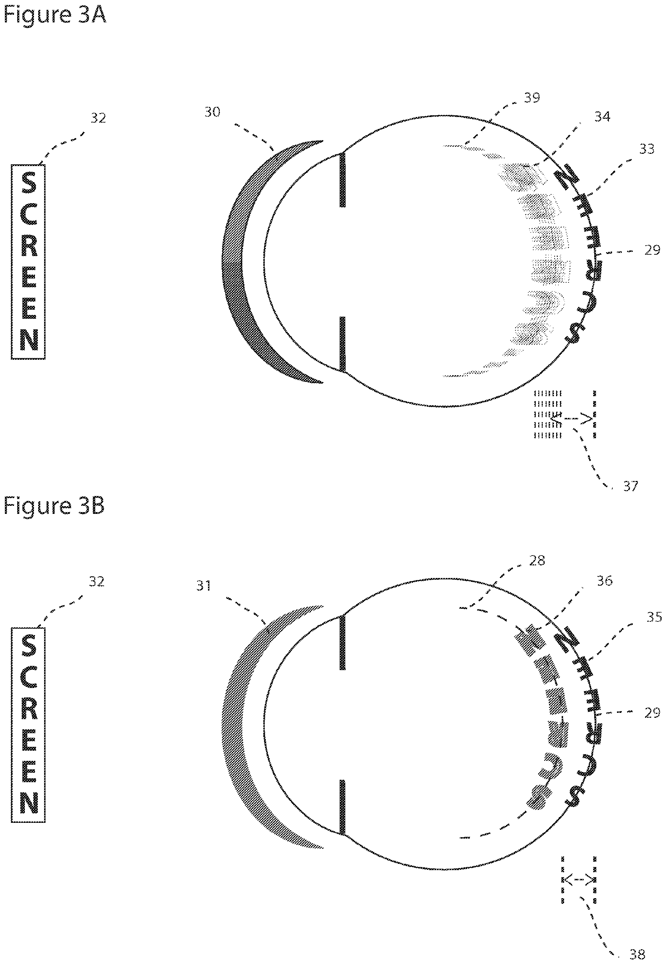

Referring to Figure 2B, the sinusoidal power profiles 20 of the defocusing zone introduces multiple defocused images that are slightly separated from each others and of lower intensity as shown in Figure 3A. The 6D power differences between correcting zones and defocusing zones ensure that defocus generated is potent enough to achieve optimal retarding effect. For example, the power of each of the defocusing zones ranges between -4 to +2D to introduce myopic defocus, if the wearer has 4D of myopia. The edge of the lens is made of a non-optic zone for lens stabilization purpose. The total lens diameter in this example is 13.6mm.

Referring to Figure 2B, the square power profile 21 of the prior art introduces a homogenous secondary defocused image. Furthermore, the prior art does not integrate any progressive transition curve as part of the power profile. Thus the power change across adjacent zones is non-continuous, abrupt, sudden, and stepwise.

In Figure 2B, the power profile of the defocusing zone is progressive and is like a sinusoidal pattern. In contrast, in Figure 2C, the prior art shows the power profile of each defocusing zone is homogenous.

In Figures 3A and 3B, a homogeneous focused image 33, 35 projected by a common object 32 is formed on the retina 29 to provide good vision.

Turning to Figure 3A, a myopic eye fitted with a concentric multi-zone multi- defocusing lens 30 has a partial sinusoidal power profile. The multiple non homogeneous defocused images 34 produced by the present multi-defocusing lens 30 are non-homogeneous and are dim compared to the focused image 33 on the retina 29. Therefore, it does not serve as a significant source of visual disturbance while a therapeutic range of myopic defocus 37 may be maintained across the retina 29.

A common object 32 is projected as a homogenous focused image 33 on the retina 29, and as multiple non homogeneous defocused images 34 in front of the retina 29. A range of defocus 37 is induced over the retina 29 both axially and peripherally as the focal planes 39 extend across the entire retina 29.

Referring to Figure 3B, a myopic eye fitted with a prior art concentric multi-zone bifocal lens 31 has a square power profile. The secondary defocused image 36 produced by the bifocal lens 31 is quite homogeneous compared to the homogenous focused image 35 on retina 29. Although a therapeutic dose of myopic defocus 38 is maintained across the retina 29, the homogeneous defocused image 36 serves as a source of visual disturbance.

A common object 32 is projected as a homogenous focused image 35 on the retina 29, and as another homogenous defocused image 36 in front of the retina 29. A homogeneous magnitude of defocus 38 is induced in front of the retina 29 both axially and peripherally as the focal plane 28 extends across the entire retina 29.

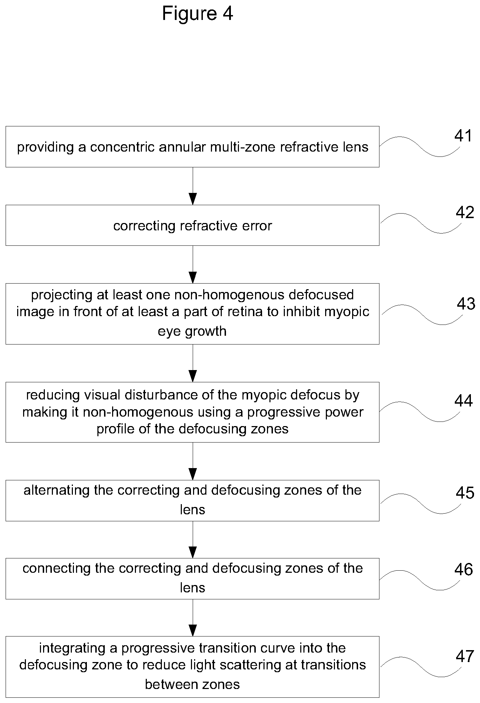

Referring to Figure 4, the method described retards the progression of myopia in a human eye. A concentric annular multi-zone refractive lens is provided (41). The lens has at least one correcting zone of optical power for correcting (42) refractive error. When the refractive error is corrected by the correcting zone, clear vision is provided for objects at all distances and natural ocular accommodation is used for near tasks.

The lens also has at least one defocusing zone for projecting (43) at least one non-homogenous defocused image in front of at least a part of retina to inhibit myopic eye growth. A non-homogenous myopic defocus is introduced on the retina using the defocusing zones at all times regardless of viewing distance. The at least one defocusing zone has at least one less negative power. The visual disturbance of the myopic defocus is reduced (44) by making it non-homogenous using a progressive power profile of the defocusing zones. By making the defocused image non-homogenous, misuse of the defocusing zones for viewing is avoided. Also, the non-homogenous myopic defocus inhibits eye growth. The correcting and defocusing zones are alternated (45) in the lens. The correcting and defocusing zones are connected (46) to each other through integrated progressive transition curves. This improves optical performance by reducing (47) light scattering at transitions between zones.

Although particular applications in curing and retarding the progression of refractive disorder of the eye have been described, it is envisaged that the present invention may be used in other applications such as preventing pathological myopic degeneration of the eye.

It will be appreciated by persons skilled in the art that numerous variations and/or modifications may be made to the invention as shown in the specific embodiments without departing from the scope of the invention as broadly described. The present embodiments are, therefore, to be considered in all respects illustrative and not restrictive.

## Claims

1. A refractive lens (9) for retarding the progression of myopia in a human eye, the refractive lens (9) comprising:
a concentric annular multi-zone refractive lens including:
correcting zones (12, 22, 24) of optical power for correcting refractive error, and
defocusing zones (23) having a progressive power profile (10) for projecting multiple defocus images or non-homogenous defocused images in front of at least a part of retina to inhibit myopic eye growth, the defocusing zones (23) having a range of less negative powers than the optical power for correcting refractive power of the correcting zones (12, 22, 24);
wherein the correcting zones (12, 22, 24) and the defocusing zones (23) are alternated in the lens,
wherein the lens has at least a total number of more than three correcting zones (12, 22, 24) and defocusing zones (23) alternating in a concentric manner,
wherein the central first correcting/defocusing zone (12, 22, 23, 24) is smaller than the pupil is measured under photopic lighting conditions,
wherein the same single homogeneous power is maintained over the correcting zones (12, 22, 24),
**characterized in that** the defocusing zones have a sinusoidal power profile.

2. The refractive lens (9) according to claim 1, wherein the lens is in the form of a refractive lens and its optical surface includes 4 to 40 alternating correcting and defocusing zones (23).

3. The refractive lens (9) according to claim 2, wherein the lens has a central zone that is a circular first correcting zone (12, 22, 24) having a diameter smaller than the pupil of the eye under photopic lighting.

4. The refractive lens (9) according to claim 3, wherein the first correcting zone (12, 22, 24) is immediately surrounded by a first defocusing zone (23) having an annular shape.

5. The refractive lens (9) according to claim 4, wherein the first defocusing zone (23) is immediately surrounded by a second correcting zone (12, 22, 24) having an annular shape.

6. The refractive lens (9) according to claim 5, wherein the second correcting zone (12, 22) is surrounded by additional defocusing zones (23) and correcting zones (12, 22, 24) in an alternating manner.

7. The refractive lens (9) according to claim 2, wherein the lens has a central zone that is a circular first defocusing zone (23) having a diameter smaller than the pupil of the eye under photopic lighting.

8. The refractive lens (9) according to claim 7, wherein the first defocusing zone (23) is immediately surrounded by a first correcting zone (12, 22, 24) having an annular shape.

9. The refractive lens (9) according to claim 8, wherein the first correcting zone (12, 22, 24) is immediately surrounded by a second defocusing zone (23) having an annular shape.

10. The refractive lens (9) according to claim 9, wherein the second defocusing zone (23) is surrounded by additional defocusing zones (23) and correcting zones (12, 22, 24) in an alternating manner.

11. The refractive lens (9) according to claim 1, wherein the maximal difference between the power of the correcting zones (12, 22, 24) and the peak power of the defocusing zones (23) is between about 0.5 to 10.0 diopters.

12. The refractive lens (9) according to claim 3 or 7, wherein the diameter of the central zone is about 2.0mm to 4.5mm.

13. The refractive lens (9) according to claim 11, wherein at least part of the defocusing zones (23) and at least a part of the correcting zone (12, 22, 24) overlies the pupil at the same time.

14. The refractive lens (9) according to claim 1, wherein the power profile of the defocusing zones (23) is progressive causing transitions between adjacent curves to become progressive.

15. The refractive lens (9) according to claim 1, wherein the progressive power profile (10) is generated by adjusting the radius of curvature of the anterior refractive surface of the lens or by adjusting the posterior curvature or the refractive index of the lens.

16. The refractive lens (9) according to claim 1, further comprising an integrated progressive transition curve between adjacent zones of the lens.

17. The refractive lens (9) according to claim 1, wherein the lens has seven correcting zones (12, 22, 24) and six defocusing zones (23) to maintain a relatively stable area ratio between the correcting zones (12, 22, 24) and defocusing zones (23) for a range of pupil sizes and lighting conditions.

18. The refractive lens (9) according to claim 1, wherein the multiple defocus images or the non-homogenous defocused images projected by the defocusing zones (23) have a lower intensity than a retinal images projected by the correcting zones (12, 22, 24).

19. The refractive lens (9) according to claim 1, wherein the correcting zones (12, 22, 24) of the lens project focused images onto the retina for visual objects positioned at all distances.

## Patentansprüche

1. Brechungslinse (9) zum Verzögern der Progression der Kurzsichtigkeit in einem menschlichen Auge, wobei die Brechungslinse (9) umfasst:
eine konzentrische, ringförmige, mehrzonige Brechungslinse umfassend:
Korrekturzonen (12, 22, 24) mit optischer Brechkraft zur Korrektur des Brechungsfehlers, und
Defokussierungszonen (23) mit einem progressiven Brechkraftprofil (10) zum Projizieren von mehrfachen defokussierten Bildern oder nicht-homogenen defokussierten Bildern vor mindestens einen Teil der Netzhaut, um das Wachstum der Kurzsichtigkeit des Auges zu hemmen, wobei die Defokussierungszonen (23) einen Bereich von weniger negativen Brechkräften als die optische Brechkraft zur Korrektur der Brechkraft der Korrekturzonen (12, 22, 24) aufweisen;
wobei die Korrekturzonen (12, 22, 24) und die Defokussierungszonen (23) in der Linse abwechselnd angeordnet sind,
wobei die Linse mindestens eine Gesamtzahl von mehr als drei Korrekturzonen (12, 22, 24) und Defokussierungszonen (23) aufweist, die sich konzentrisch abwechseln,
wobei die zentrale erste Korrektur-/Defokussierungszone (12, 22, 23, 24) kleiner ist als die Pupille unter photopischen Beleuchtungsbedingungen gemessen wird,
wobei die gleiche einzelne homogene Brechkraft über die Korrekturzonen (12, 22, 24) beibehalten wird,
**dadurch gekennzeichnet, dass** die Defokussierungszonen ein sinusförmiges Brechkraftprofil aufweisen.

2. Brechungslinse (9) nach Anspruch 1, wobei die Linse die Form einer Brechungslinse aufweist und ihre optische Oberfläche 4 bis 40 abwechselnde Korrektur- und Defokussierungszonen (23) aufweist.

3. Brechungslinse (9) nach Anspruch 2, wobei die Linse eine zentrale Zone aufweist, die eine kreisförmige erste Korrekturzone (12, 22, 24) ist, die einen Durchmesser aufweist, der kleiner ist als die Pupille des Auges bei photopischer Beleuchtung.

4. Brechungslinse (9) nach Anspruch 3, wobei die erste Korrekturzone (12, 22, 24) unmittelbar von einer ersten Defokussierungszone (23) mit Ringform umgeben ist.

5. Brechungslinse (9) nach Anspruch 4, wobei die erste Defokussierungszone (23) unmittelbar von einer zweiten Korrekturzone (12, 22, 24) mit Ringform umgeben ist.

6. Brechungslinse (9) nach Anspruch 5, wobei die zweite Korrekturzone (12, 22) abwechselnd von weiteren Defokussierungszonen (23) und Korrekturzonen (12, 22, 24) umgeben ist.

7. Brechungslinse (9) nach Anspruch 2, wobei die Linse eine zentrale Zone aufweist, die eine kreisförmige erste Defokussierungszone (23) ist, die einen Durchmesser aufweist, der kleiner ist als die Pupille des Auges bei photopischer Beleuchtung.

8. Brechungslinse (9) nach Anspruch 7, wobei die erste Defokussierungszone (23) unmittelbar von einer ersten Korrekturzone (12, 22, 24) mit Ringform umgeben ist.

9. Brechungslinse (9) nach Anspruch 8, wobei die erste Korrekturzone (12, 22, 24) unmittelbar von einer zweiten Defokussierungszone (23) mit Ringform umgeben ist.

10. Brechungslinse (9) nach Anspruch 9, wobei die zweite Defokussierungszone (23) abwechselnd von weiteren Defokussierungszonen (23) und Korrekturzonen (12, 22, 24) umgeben ist.

11. Brechungslinse (9) nach Anspruch 1, wobei die maximale Differenz zwischen der Brechkraft der Korrekturzonen (12, 22, 24) und der Spitzenbrechkraft der Defokussierungszonen (23) zwischen etwa 0,5 bis 10,0 Dioptrien beträgt.

12. Brechungslinse (9) nach Anspruch 3 oder 7, wobei der Durchmesser der zentralen Zone etwa 2,0 mm bis 4,5 mm beträgt.

13. Brechungslinse (9) nach Anspruch 11, wobei zumindest ein Teil der Defokussierungszonen (23) und zumindest ein Teil der Korrekturzone (12, 22, 24) gleichzeitig die Pupille überlagern.

14. Brechungslinse (9) nach Anspruch 1, wobei das Brechkraftprofil der Defokussierungszonen (23) progressiv ist, wodurch die Übergänge zwischen benachbarten Kurven progressiv werden.

15. Brechungslinse (9) nach Anspruch 1, wobei das progressive Brechkraftprofil (10) durch Einstellen des Krümmungsradius der vorderen Brechungsfläche der Linse oder durch Einstellen der hinteren Krümmung oder des Brechungsindex der Linse erzeugt wird.

16. Brechungslinse (9) nach Anspruch 1, die ferner eine integrierte progressive Übergangskurve zwischen benachbarten Zonen der Linse umfasst.

17. Brechungslinse (9) nach Anspruch 1, wobei die Linse sieben Korrekturzonen (12, 22, 24) und sechs Defokussierungszonen (23) aufweist, um ein relativ stabiles Flächenverhältnis zwischen den Korrekturzonen (12, 22, 24) und den Defokussierungszonen (23) für einen Bereich von Pupillengrößen und Beleuchtungsbedingungen beizubehalten.

18. Brechungstinse (9) nach Anspruch 1, wobei die von den Defokussierungszonen (23) projizierten mehrfach defokussierten Bilder oder die nicht homogen defokussierten Bilder eine geringere Intensität aufweisen als ein von den Korrekturzonen (12, 22, 24) projiziertes Netzhautbild.

19. Brechungslinse (9) nach Anspruch 1, wobei die Korrekturzonen (12, 22, 24) der Linse für Sehobjekte in allen Entfernungen fokussierte Bilder auf die Netzhaut projizieren.

## Revendications

1. Lentille réfractive (9) pour retarder la progression de la myopie dans un oeil humain, la lentille réfractive (9) comprenant :
une lentille réfractive multizone annulaire concentrique comprenant :
des zones de correction (12, 22, 24) de la réfringence optique pour corriger l'erreur de réfraction, et
des zones de défocalisation (23) ayant un profil de réfringence progressive (10) pour projeter des images défocalisées multiples ou des images défocalisées non homogènes devant au moins une partie de la rétine pour inhiber la croissance de l'oeil myope, les zones de défocalisation (23) ayant une gamme de réfringences négatives inférieures à la réfringence optique pour la correction de la réfringence des zones de correction (12, 22, 24) ;
dans laquelle les zones de correction (12, 22, 24) et les zones de défocalisation (23) sont alternées dans la lentille,
dans laquelle la lentille a au moins un nombre total de plus de trois zones de correction (12, 22, 24) et zones de défocalisation (23) alternant de manière concentrique,
dans laquelle la première zone centrale de correction/défocalisation (12, 22, 23, 24) est plus petite que la pupille est mesurée dans des conditions d'éclairage photopique,
où la même réfringence homogène unique est maintenue sur les zones de correction (12, 22, 24),
**caractérisée en ce que** les zones de défocalisation ont un profil de réfringence sinusoïdal.

2. Lentille réfractive (9) selon la revendication 1, dans laquelle la lentille se présente sous la forme d'une lentille réfractive et sa surface optique comprend de 4 à 40 zones de correction et de défocalisation alternées (23).

3. Lentille réfractive (9) selon la revendication 2, dans laquelle la lentille a une zone centrale qui est une première zone de correction circulaire (12, 22, 24) ayant un diamètre inférieur à la pupille de l'oeil sous éclairage photopique.

4. Lentille réfractive (9) selon la revendication 3, dans laquelle la première zone de correction (12, 22, 24) est immédiatement entourée par une première zone de défocalisation (23) de forme annulaire.

5. Lentille réfractive (9) selon la revendication 4, dans laquelle la première zone de défocalisation (23) est immédiatement entourée d'une deuxième zone de correction (12, 22, 24) de forme annulaire.

6. Lentille réfractive (9) selon la revendication 5, dans laquelle la deuxième zone de correction (12, 22) est entourée de zones de défocalisation (23) et de zones de correction (12, 22, 24) supplémentaires de manière alternée.

7. Lentille réfractive (9) selon la revendication 2, dans laquelle la lentille a une zone centrale qui est une première zone de défocalisation circulaire (23) ayant un diamètre inférieur à la pupille de l'œil sous éclairage photopique.

8. Lentille réfractive (9) selon la revendication 7, dans laquelle la première zone de défocalisation (23) est immédiatement entourée d'une première zone de correction (12, 22, 24) de forme annulaire.

9. Lentille réfractive (9) selon la revendication 8, dans laquelle la première zone de correction (12, 22, 24) est immédiatement entourée par une deuxième zone de défocalisation (23) de forme annulaire.

10. Lentille réfractive (9) selon la revendication 9, dans laquelle la deuxième zone de défocalisation (23) est entourée de zones de défocalisation (23) et de zones de correction (12, 22, 24) supplémentaires de manière alternée.

11. Lentille réfractive (9) selon la revendication 1, dans laquelle la différence maximale entre la réfringence des zones de correction (12, 22, 24) et la réfringence maximale des zones de défocalisation (23) est entre environ 0,5 et 10,0 dioptries.

12. Lentille réfractive (9) selon la revendication 3 ou 7, dans laquelle le diamètre de la zone centrale est d'environ 2,0 mm à 4,5 mm.

13. Lentille réfractive (9) selon la revendication 11, dans laquelle au moins une partie des zones de défocalisation (23) et au moins une partie de la zone de correction (12, 22, 24) recouvrent en même temps la pupille.

14. Lentille réfractive (9) selon la revendication 1, dans laquelle le profil de réfringence des zones de défocalisation (23) est progressif, ce qui fait que les transitions entre les courbes adjacentes deviennent progressives.

15. Lentille réfractive (9) selon la revendication 1, dans laquelle le profil de réfringence progressive (10) est généré en ajustant le rayon de courbure de la surface réfractive antérieure de la lentille ou en ajustant la courbure postérieure ou l'indice de réfraction de la lentille.

16. Lentille réfractive (9) selon la revendication 1, comprenant en outre une courbe de transition progressive intégrée entre les zones adjacentes de la lentille.

17. Lentille réfractive (9) selon la revendication 1, dans laquelle la lentille a sept zones de correction (12, 22, 24) et six zones de défocalisation (23) pour maintenir un rapport de surface relativement stable entre les zones de correction (12, 22, 24) et les zones de défocalisation (23) pour une gamme de tailles de pupilles et de conditions d'éclairage.

18. Lentille réfractive (9) selon la revendication 1, dans laquelle les images défocalisées multiples ou les images défocalisées non homogènes projetées par les zones de défocalisation (23) ont une intensité inférieure à celle des images rétiniennes projetées par les zones de correction (12, 22, 24).

19. Lentille réfractive (9) selon la revendication 1, dans laquelle les zones de correction (12, 22, 24) de la lentille projettent des images focalisées sur la rétine pour des objets visuels positionnés à toutes distances.
